# EUROPEAN PATENT SPECIFICATION

(11) **EP 2 554 677 B1**
(45) Date of publication and mention of the grant of the patent: **29.04.2015**
(21) Application number: 11759568.6
(22) Date of filing: 25.03.2011
(51) Int. Cl.: C12Q 1/06, C12N 1/20

(54) **VIABLE BACTERIA COUNT MEASUREMENT METHOD AND CULTURE MEDIUM**
VERFAHREN ZUR MESSUNG EINER ANZAHL LEBENSFÄHIGER BAKTERIEN UND KULTURMEDIUM DAFÜR
PROCÉDÉ DE MESURE D'UN NOMBRE DE BACTÉRIES VIABLES, ET MILIEU DE CULTURE

(30) Priority: 26.03.2010 JP 2010072369
(43) Date of publication of application: 06.02.2013
(73) Proprietor: Morinaga Milk Industry Co., Ltd., Minato-ku Tokyo 108-8384 (JP)
(72) Inventor: MUTO Masamichi, Zama-shi Kanagawa 252-8583 (JP); ABE Fumiaki, Zama-shi Kanagawa 252-8583 (JP); YAESHIMA Tomoko, Zama-shi Kanagawa 252-8583 (JP)
(74) Representative: Vossius & Partner Patentanwälte Rechtsanwälte mbB
(86) International application number: PCT/JP2011/057335
(87) International publication number: WO 2011/118764

(56) References cited:
- PLUMMER ET AL: "Monitoring source water for microbial contamination: Evaluation of water quality measures", WATER RESEARCH, ELSEVIER, AMSTERDAM, NL, vol. 41, no. 16, 4 August 2007 (2007-08-04), pages 3716-3728, XP022182700, ISSN: 0043-1354, DOI: 10.1016/J.WATRES.2007.05.004
- LONG, S.C. ET AL.: 'An optimized enumeration method for sorbitol-fermenting Bifidobacteria in water samples.' CAN. J. MICROBIOL. vol. 51, no. 5, 2005, pages 413 - 422, XP008154841
- MITSUOKA, T.: 'Recent Trends in Research on Intestinal Flora.' BIFIDOBACTERIA MICROFLORA vol. 1, no. 1, 1982, pages 3 - 24, XP008155156
- ROY, D. ET AL.: 'Evaluation of rapid methods for differentiation of Bifidobacterium species.' J. APPL. BACTERIOL. vol. 69, no. 5, 1990, pages 739 - 749, XP008154304
- SILVI, S. ET AL.: 'An assessment of three selective media for bifidobacteria in faeces.' J. APPL. BACTERIOL. vol. 81, no. 5, 1996, pages 561 - 564, XP008154849
- MARA, D.D. ET AL.: 'Sorbitol-fermenting bifidobacteria as specific indicators of human faecal pollution.' J. APPL. BACTERIOL. vol. 55, no. 2, 1983, pages 349 - 357, XP008154842
- RHODES, M.W. ET AL.: 'Sorbitol-fermenting bifidobacteria as indicators of diffuse human faecal pollution in estuarine watersheds.' J. APPL. MICROBIOL. vol. 87, no. 4, 1999, pages 528 - 535, XP008154848
- BONJOCH, X. ET AL.: 'Enumeration of bifidobacterial populations with selective media to determine the source of waterborne fecal pollution.' WATER RES. vol. 39, no. 8, 2005, pages 1621 - 1627, XP008154846

## Description

The present invention relates to a measurement method for the viable cell count of the breve species alone, amongst the bacterial cells tested which include bifidobacteria, through a culture method, and also to a culture medium which is useful as a selective medium for the aforementioned measurement method.

Priority is claimed on Japanese Patent Application No. 2010-72369, filed March 26, 2010.

### [Background Art]

Bifidobacteria are widely known as one of the useful group of intestinal bacteria and a multitude of publications exist relating to the physiological significance of this group of bacteria. For example, it has become clear that bifidobacteria produce organic acids such as lactic acid and acetic acid within the intestine, and also have effects in terms of suppressing the multiplication of harmful bacteria, producing vitamins, and activating immunity and the like.

For this reason, various preparations containing live bifidobacterial cells have been proposed in the past (Non-Patent Document 1). In addition, for the purpose of maintaining good health through the ingestion of bifidobacteria, various food products have been developed that contain bifidobacteria, such as fermented milk products including yogurts, sweets, drinks, and health foods. Furthermore, infants who are fed with breast milk during their infancy and early childhood tend to have superior levels of bifidobacteria, and therefore products such as powdered milk for infants or children containing bifidobacteria and/or lactic acid bacteria are also being developed in countries outside Japan. Currently, the longum species are mainly added as bifidobacterial species to food products in the world market, and there are also some products which use the breve species in combination (Non-Patent Document 2).

It is thought that individual display of the viable cell count for useful bacteria added in the products is beneficial for consumers in terms of providing as much information on the products as possible. In addition, with respect to the display of viable cell counts, the law has been enacted in Indonesia to display the viable cell count for each useful bacterial species added to the products.

Currently, as a method for determining the viable cell count for either one of bifidobacteria and lactic acid bacteria in the products containing both bacterial species, a method to use a culture medium in which only bifidobacteria are possible to grow or a method to culture lactic acid bacteria alone through aerobic cultivation has already been established.

In addition, as a method for identifying the species of bifidobacteria in the products containing several species of bifidobacteria or lactic acid bacteria and also determining the viable cell count thereof, there is a method in which they are determined either from the morphology (i.e., color, shape, or the like) of colonies formed anaerobically or from the morphology of bacteria through Gram staining, by growing bacteria streaked on a culture medium that is prepared by adding sterile defibrinated blood to a BL agar medium (Non-Patent Document 3).

Non-Patent Document 4 describes an enumeration method for sorbitol-fermenting Bifidobacteria in water samples using different media.

### [Citation List]

### [Non-Patent Documents]

[Non-Patent Document 1] "Research on bifidobacteria", written and edited by Tomotari Mitsuoka, published by Japan Bifidus Foundation (1994), pages 266 to 267
[Non-Patent Document 2] "Research on bifidobacteria", written and edited by Tomotari Mitsuoka, published by Japan Bifidus Foundation (1994), pages 282 to 283
[Non-Patent Document 3] "Method for the enumeration ofbifidobacteria in fermented milks and fermented milk drinks", published by the Bifidobacterium Testing Methods Review Committee of the Japanese Association of Fermented Milks and Fermented Milk Drinks, March 2000, pages 1 to 13
[Non-Patent Document 4] "An optimized enumeration method for sorbitol-fermenting Bifidobacteria in water samples" by Long SC et al., Can J Microbiol., 2005, 51(5), pages 413 to 422

### [Summary of Invention]

### [Technical Problem]

However, in those cases where the morphologies of colonies or the shapes of bacteria are similar, specialized knowledge is required to determine the bacterial species with the method as described in the above Non-Patent Document 3. For this reason, it is sometimes very difficult to determine the viable cell count for each bacterial species. In addition, there are many foreign countries where it is impossible to obtain sterile defibrinated blood. In those countries, it is impossible to prepare a BL agar medium containing blood, and thus the above method cannot be employed.

Currently, because there are products containing both the breve species and the longum species as described above, and also because the breve species, the longum species, the lactis species and the like are widely used in the world probiotic market, a technique has been required which can easily identify specific bacterial species such as the breve species through a culture method in the products where several bifidobacterial species coexist.

The present invention aims to address the problems outlined above, and has an object of providing a measurement method capable of easily determining the viable cell count for the breve species alone, amongst the bacterial cells tested which include bifidobacteria, by using a specific culture method; and a culture medium which is useful as a selective medium for the above measurement method and which is also easy to prepare.

### [Solution to Problem]

In order to solve the above problems, the inventors of the present invention have conducted detailed studies on the sugars and saccharides listed in Tables 15.51 and 15.54 in Bergey's Manual of Systematic Bacteriology (1986, vol. 2, page 1,428), and sugars and saccharides listed in "Recent Trends in Research on Intestinal Flora" (written by Tomotari Mitsuoka, in Bifidobacteria Microflora, vol. 1 (1), pp. 3-24). As a result, it was found that amongst the breve species, the longum species, the animalis species, and the infantis species, most bacterial cells of breve species may assimilate sorbitol and mannitol during the viable cell count through a culture method; and that amongst the longum species, the animalis species, and the infantis species, only a very small proportion of strains of the infantis species was capable of assimilating mannitol and sorbitol. The inventors of the present invention have conducted intensive and extensive investigation based on these discoveries. As a result, they have succeeded in developing a culture medium, within which the breve species alone are capable of forming large colonies, by including mannitol and/or sorbitol as a sole sugar source and adjusting the concentrations of medium components other than sugars (such as proteins and peptides) to a certain range. In other words, the inventors of the present invention have used the assimilation capacity of breve species for mannitol and sorbitol and limited the concentrations of medium components, thereby succeeding in developing a culture medium that reduces the colony forming capacity of other bifidobacterial species and enables determination of viable cell count for the breve species alone. In this manner, it became possible to determine the viable cell count for breve species alone, amongst the aforementioned four bacterial species.

The measurement method and the culture medium according to the present invention that achieve the object described above include the following aspects.
[1] A method for measuring a viable cell count of a microorganism belonging to Bifidobacterium breve alone, amongst bacteria to be tested which include a microorganism belonging to the genus Bifidobacterium, through the use of a culture medium, the method characterized in that the aforementioned culture medium meets the following requirements 1) and 2), and the aforementioned viable cell count is determined by measuring the colonies having a diameter of 0.7 mm or larger which are formed in the aforementioned culture medium:
   1) contains at least one sugar alcohol selected from sorbitol and mannitol as a sole sugar source, wherein a concentration of the aforementioned sugar alcohol within the aforementioned culture medium is not more than 4% by mass, relative to the total mass of the culture medium; and
   2) contains peptone, meat extract and yeast extract as nitrogen sources, wherein a content of the aforementioned peptone is 6.0 to 14.0 g/1,000 mL, a content of the aforementioned meat extract is 6.0 to 14.0 g/1,000 mL, and a content of the aforementioned yeast extract is 1.8 to 4.2 g/1,000 mL, in 1,000 mL of the aforementioned culture medium.
[2] The method according to the above aspect [1] in which the aforementioned microorganism belonging to Bifidobacterium breve is a Bifidobacterium breve M-16V strain.
[3] The method according to the above aspect [1] or [2] in which the aforementioned bacteria to be tested include, as the aforementioned microorganism belonging to the genus Bifidobacterium, the aforementioned microorganism belonging to Bifidobacterium breve and at least one type of microorganism selected from the group consisting of a microorganism belonging to Bifidobacterium longum subsp. longum, a microorganism belonging to Bifidobacterium animalis subsp. lactis, and a microorganism belonging to Bifidobacterium longum subsp. infantis.
[4] The method according to the above aspect [3] in which the aforementioned microorganism belonging to Bifidobacterium longum subsp. longum is a Bifidobacterium longum subsp. longum BB536 strain.
[5] A culture medium for measuring a viable cell count of a microorganism belonging to Bifidobacterium breve alone, amongst bacteria to be tested which include a microorganism belonging to the genus Bifidobacterium, which is used in the method described in any one of the above aspects [1] to [4], wherein the aforementioned culture medium satisfies the following requirements 1) to 5):
   1) contains at least one sugar alcohol selected from sorbitol and mannitol as a sole sugar source;
   2) a concentration of the aforementioned sugar alcohol is not more than 4% by mass, relative to the total mass of the culture medium;
   3) contains yeast extract, within 1,000 mL of the culture medium, at a concentration from 1.8 to 4.2 g/1,000 mL;
   4) contains meat extract, within 1,000 mL of the culture medium, at a concentration from 6.0 to 14.0 g/1,000 mL; and
   5) contains peptone, within 1,000 mL of the culture medium, at a concentration from 6.0 to 14.0 g/1,000 mL.

In the description and the claims of the present invention, concentration (%) values represent w/v (mass/volume) values, unless otherwise specified.

In this description, "bifidobacterium" refers to a microorganism which belongs to the genus Bifidobacterium.

In addition, the breve species refers to a microorganism which belongs to Bifidobacterium breve, the longum species refers to a microorganism which belongs to Bifidobacterium longum subsp. longum, the animalis species refers to a microorganism which belongs to Bifidobacterium animalis, the lactis species refers to a microorganism which belongs to Bifidobacterium animalis subsp. lactis and the infantis species refers to a microorganism which belongs to Bifidobacterium longum subsp. infantis.

The term "bacteria powder" refers to bacteria that has been converted to a powdered form.

### [Advantageous Effects of Invention]

According to the present invention, there are provided a measurement method capable of easily measuring the viable cell count of breve species alone, amongst the bacterial cells tested which include bifidobacteria, by using a specific culture method; and a culture medium which is useful as a selective medium for the above measurement method and which is also easy to prepare.

### [Description of Embodiments]

In the measurement method according to the present invention, the viable cell count of the breve species alone is determined, amongst the bacterial cells tested which include bifidobacteria, by using a culture medium that contains at least one sugar alcohol selected from sorbitol and mannitol as a sole sugar source, as further defined in the claims.

The culture medium will be described later in detail.

There are no particular limitations on the bacterial strain of breve species, and the bacterial strain may be a deposited strain from a public microorganism depository which has been deposited in the public culture collections (such as ATCC, NTCC, JCM, DSMZ, BCCM and LMG) to date as a strain belonging to the breve species, or may be a strain isolated from nature by known methods. Examples of the deposited strain from a public microorganism depository include the ATCC 15700^{T} strain and the M-16V strain (deposited under the Deposition Number: BCCM/LMG23729 and commercially available from Morinaga Milk Industry Co., Ltd. as the M-16V bacteria powder). The superscript T denotes a type strain.

The bifidobacteria included in the bacteria to be tested may consist of the breve species alone. However, in view of the usefulness of the present invention, in addition to the breve species, it is preferable to include at least another type of bifidobacteria other than the breve species.

There are no particular limitations on the aforementioned another type of bifidobacteria other than the breve species, and examples thereof include bifidobacteria that are widely used in general as probiotic bacteria. Of these, it is preferable to include at least one type of species selected from the longum species, the lactis species and the infantis species, since the colonies they form on the aforementioned culture medium have small sizes and can be easily distinguished from the colonies formed by the breve species. Among them, it is preferable to include at least one type of strain selected from the ATCC 15707^{T} strain (longum species), the BB536 strain (longum species, deposited under the Deposition Number: ATCC BAA-999 and commercially available from Morinaga Milk Industry Co., Ltd. as the BB536 bacteria powder), the DSM 10140^{T} strain (lactis species) and the ATCC 15697^{T} strain (infantis species), and it is particularly desirable to include the BB536 strain (longum species).

There are no particular restrictions on the types of samples containing bacteria to be tested which can be measured for viable cell count using the present invention, provided the sample contains bacteria to be tested. Specific examples thereof include various food products such as fermented milk products, sweets, drinks, health foods and powdered milk for infants or children, products such as drugs and livestock feed, and other products prepared by processing the above products through grinding, dilution with a diluent, or the like.

With respect to the incubation of the aforementioned sample, apart from the use of the culture medium according to the present invention as a culture medium, known culture methods which have been conventionally used to determine the viable cell count of microorganisms through culture methods can be employed. Examples of the aforementioned culture methods include a solid culture method (namely, a culture method in which culturing is performed on an agar medium).

Specific examples of the solid culture method include the pour plate method, the spread plate method and the spiral plate method. The pour plate method is a method in which a test sample (i.e., a sample or a diluted material prepared by diluting the sample with a diluent) is mixed with a heated and melted agar medium, and the mixture is then cooled, solidified and cultured. The spread plate method is a method in which a test sample is smeared across the top of an agar medium and then cultured. The spiral plate method is a method in which a test sample is plated on a culture medium with a concentration gradient using an instrument or the like.

More specifically, the measurement method according to the present invention can be carried out in the following manner. A test sample (i.e., a sample or a diluted material prepared by diluting the sample with a diluent) is cultured with the culture medium according to the present invention which contains agar to a concentration of about 1.5% by mass, and the number of colonies with a predetermined size or larger (for example, those having a diameter of 0.7 mm or more) among the formed colonies is counted. The number of colonies counted at this time corresponds with the viable cell count of the breve species contained within the tested sample cultured on the aforementioned agar medium. Accordingly, the viable cell count (/g) for the breve species contained in the sample can be determined from this value and the dilution ratio.

There are no particular limitations on the diluent, and known diluents such as physiological saline and the diluent (A) described in the above-mentioned Non-Patent Document 3 (the anaerobic sample diluent disclosed in "Standard Methods of Analysis in Food Safety Regulation") can be used.

In terms of the culture conditions in the present invention, conventional culture conditions may be employed as a condition for culturing the breve species.

The number of colonies are usually counted through visual inspection after 48 hours of incubation at 37°C under anaerobic conditions.

The culture medium used in the aforementioned measurement method according to the present invention contains at least one sugar alcohol selected from sorbitol and mannitol (hereafter, sometimes referred to as a specific sugar source) as a sole sugar source.

The aforementioned specific sugar source may consist of sorbitol alone or mannitol alone, or may be a combination of sorbitol and mannitol. In addition, sorbitol is preferably D-sorbitol, and mannitol is preferably D-mannitol. Alternatively, a combination of D-sorbitol and D-mannitol may be used.

In the above culture medium, from the viewpoint of selection for the breve species, the concentration of the aforementioned specific sugar source (i.e., the concentration of sorbitol or mannitol) is not more than 4% by mass, relative to the total mass of the culture medium. If the effects on osmotic pressure or economic factors are taken into consideration, then the above concentration is preferably from 1 to 4% by mass, and more preferably from 1 to 3% by mass.

It should be noted that examples of the sugar source other than the aforementioned specific sugar source that are not included in the culture medium of the present invention include glucose, starch, sucrose, raffinose, galactose and arabinose.

The culture medium may contain a component other than the aforementioned specific sugar source. Hereafter, the components that constitute the aforementioned culture medium, other than the aforementioned specific sugar source serving as a sole sugar source, will be described as basal medium components.

Examples of the basal medium components include nitrogen sources such as yeast extract, meat extract and peptone; and other salts including sodium salts such as sodium chloride, sodium acetate and sodium propionate, L-cysteine hydrochlorides, phosphates and sulfates (such as magnesium sulfate and manganese sulfate). Among these, the culture medium used in the measurement method of the present invention contains yeast extract, meat extract and peptone as nitrogen sources.

As the yeast extract, meat extract and peptone, each of those that is generally used for the cultivation of microorganisms can be used.

Yeast extract is a source of nutrition produced using yeasts as a raw material which is moderately broken down by autolytic enzymes, and specific examples thereof include the Yeast Extract product (manufactured by Becton, Dickinson and Company). Meat extract is a source of nutrition obtained from the meat exudate which complements the properties of peptone nutrition by providing essential elements such as minerals, phosphoric acid and energy sources that are absent in peptone. Specific examples thereof include the 'LAB-MEMCO' powder (manufactured by Oxoid Ltd.). Peptone is prepared by partial hydrolysis of milk casein, meat, soy protein or the like with a proteolytic enzyme or an acid. Specific examples thereof include Bacto^{™} peptone (manufactured by Becton, Dickinson and Company).

The content of yeast extract is 1.8 to 4.2 g/1,000 mL, and preferably 2.7 to 4.2 g/1,000 mL, in 1,000 mL of the culture medium.

The content of meat extract in 1,000 mL of the culture medium is 6.0 to 14.0 g/1,000 mL, and preferably 9.0 to 14.0 g/1,000 mL.

The content of peptone in 1,000 mL of the culture medium is 6.0 to 14.0 g/1,000 mL, and preferably 9.0 to 14.0 g/1,000 mL.

Each of the above contents of yeast extract, meat extract and peptone (g/1,000 mL) indicate the solid content (g) included in 1,000 mL of the culture medium.

From the viewpoint of osmotic pressure, the culture medium used in the present invention preferably contains the aforementioned salts. As the aforementioned salts, sodium salts are preferred, and sodium chloride and sodium acetate are particularly desirable.

The content of the aforementioned salts in 1,000 mL of the culture medium is preferably 5 to 15 g/1,000 mL.

It is particularly advantageous that the culture medium used in the present invention satisfies all of the following requirements 1) to 5) because the breve species alone easily form large colonies, and thus the viable cell count for the breve species alone can be easily determined accurately from those where several Bifidobacteria species coexist:
1) contains the aforementioned specific sugar source as a sole sugar source;
2) a concentration of the aforementioned specific sugar source is not more than 4% by mass, relative to the total mass of the culture medium;
3) contains yeast extract, within 1,000 mL of the culture medium, at a concentration from 1.8 to 4.2 g/1,000 mL;
4) contains meat extract, within 1,000 mL of the culture medium, at a concentration from 6.0 to 14.0 g/1,000 mL; and
5) contains peptone, within 1,000 mL of the culture medium, at a concentration from 6.0 to 14.0 g/1,000 mL.

The culture medium according to the present invention may also include other components besides the above-mentioned components, if necessary, provided that these other components do not impair the effects of the present invention.

There are no particular limitations on these other components, and the types of components typically added to a culture medium may be used. For example, agar is added in those cases where the solid culture method is employed as a culture method in the measurement of the viable cell count. Agar is usually added so that the final concentration thereof achieved within the culture medium is about 1.5% by mass.

Furthermore, any antibiotic can be added thereto, provided that the growth of colonies and the viable cell count of the breve species are not adversely affected.

By using a culture medium containing the aforementioned specific sugar source for which the breve species exhibit the mode of assimilation as a sole sugar source as described above, it is possible to identify the breve species alone and to easily measure the viable cell count thereof in the products where several types of bifidobacteria including the breve species coexist. In other words, only the breve species form large colonies in such a culture medium.

For example, even the colonies formed by some of the strains of infantis species that exhibit similar assimilation mode for the aforementioned specific sugar source in the culture medium according to the present invention have smaller sizes than those formed by the breve species.

The size of the colonies become even smaller in the cases of bifidobacterial species (such as the longum species and lactis species) with no assimilation capacity for the aforementioned specific sugar source. It is thought that the reason for causing this difference in colony size is due to the aforementioned composition of the culture medium which reduces the colony forming capacity of the bifidobacterial species other than the breve species.

For this reason, when the products where several types ofbifidobacteria including the breve species coexist are cultured using the culture medium of the present invention, it is possible to identify the breve species alone and to measure the viable cell count thereof by counting the number of colonies grown to a large size, which is simple and easy, without examining the shapes of colonies and bacteria. In addition, the measured values are about as accurate as those obtained with existing culture media, such as the aforementioned culture medium prepared by adding sterile defibrinated blood to the BL agar medium.

Therefore, the culture medium of the present invention is useful as a selective medium for measuring the viable cell count of breve species within the product where several types ofbifidobacteria including the breve species coexist, through a culture method.

Moreover, it is also easy to prepare the culture medium according to the present invention since the materials that are difficult to obtain such as sterile defibrinated blood are not required.

In obtaining the above effects, it is important that each of the components serving as nitrogen sources (namely peptone, meat extract and yeast extract) is combined at predetermined concentrations.

Note that among the salts which can be included within the culture medium of the present invention, the contents of magnesium sulfate and manganese sulfate are preferably as low as possible, in order to improve the colony growth. More specifically, the content of magnesium sulfate in 1,000 mL of the culture medium is preferably not more than 0.1 g/1,000 mL, and most preferably 0 g/1,000 mL. The content of manganese sulfate is preferably not more than 0.05 g/1,000 mL, and most preferably 0 g/1,000 mL.

There are no particular limitations on the method for preparing the culture medium, and the medium can be prepared using known methods.

More specifically, first, optional components such as agar, sodium chloride, sodium acetate and L-cysteine hydrochloride together with water are added and dissolved in the mixture of yeast extract, meat extract and peptone to prepare a basal medium. The concentrations of each component in the basal medium at this time point are adjusted to a level of about 1.25 times as high as the final concentrations thereof. Next, the above basal medium is sterilized for 15 minutes at 121°C in an autoclave.

A sugar solution is prepared separately by dissolving the aforementioned specific sugar source in water. The concentration of sugar in the sugar solution at this time point is adjusted to a level of about 5 times as high as the final concentration thereof. Next, the sugar solution is sterilized by filter sterilization.

Then, the basal medium and the sugar solution are mixed at a ratio of 4:1 (volume ratio), thereby yielding a culture medium containing each component at a desired concentration. In this description of the present invention, 1,000 mL of the culture medium refers to a culture medium which is prepared by diluting the basal medium components and sugars with water to make up a total volume of 1,000 mL.

It should be noted that in the present invention, the culture medium used in the method for measuring the viable cell count of the breve species alone amongst the bacteria to be tested including bifidobacteria can also be described as a selective medium for the breve species, a selective medium for the breve species that contains at least one sugar alcohol selected from sorbitol and mannitol as a sole sugar source, or the like.

### [Examples]

A more detailed description of the present invention is presented below based on a series of test examples and examples, although the present invention is in no way limited by the following examples.

### <Test Example 1: Study on basal medium>

The studies on the active ingredient concentrations in the basal medium were conducted through use of the media prepared by employing a basal medium A or B which contained each of the basal medium components listed in Table 1, and adjusting the concentration (1.0-fold concentration) of each component in 1000 mL of the respective basal media to concentrations indicated in Tables 2 and 3. The concentration ratio indicated in Tables 2 and 3 represents a ratio of change in the concentration of each basal medium component in 1,000 mL of the basal medium, based on the contents of each basal medium component listed in Table 1 (set as 1.0-fold), while maintaining the mixing ratio listed in Table 1.

In general, the Reinforced Clostridial Medium Agar (RCA medium), the modified de Man Rogosa and Sharpe (mMRS) medium prepared by adding L-cysteine hydrochloride to the MRS medium, or the like is used for the determination of viable cell count of bifidobacteria. Accordingly, based on the medium compositions of RCA medium and mMRS medium, the basal medium A and basal medium B which contained each basal medium component listed in Table 1 were prepared.

Agar was added to each of the above basal media so that the final concentration thereof (i.e., concentration following the addition of D-mannitol or D-sorbitol) was 1.5% by mass, relative to the total mass of the culture medium, and D-mannitol or D-sorbitol was further added thereto so that the final concentration thereof was 1% by mass, relative to the total mass of the culture medium, thereby preparing the respective culture media 1-1 to 1-14 and 2-1 to 2-8.

The viable cell count through a culture method (pour plate method) was carried out by the following procedure using the prepared culture media.

As the test sample, the M-16V bacteria powder (manufactured by Morinaga Milk Industry Co., Ltd., a product containing 1.7 × 10¹¹ cells/g) was used. A 0.85% physiological saline solution was used as a diluent for diluting the test sample during the viable cell count.

The test sample was diluted with the diluent to prepare a measurement sample. The measurement sample was poured onto a plate through a pour plate method together with the culture medium which was dissolved in advance by heating to 45°C. After the solidification of agar, incubation at 37°C under anaerobic conditions was performed for 48 hours.

Following the incubation, the number of colonies that had grown on the culture medium was measured through visual inspection, and the number of viable bacterial cells within the test sample was calculated from this measured value. The results are shown in Tables 2 and 3. In addition, the diameters (mm) of the colonies were measured using a 0.1 mm scale, and the average value thereof was determined. The average value determined as described above is indicated in Tables 2 and 3 as the "colony diameter".

In tables, the expression "%" denotes "% by mass".

**[Table 1]**

| Basal medium component | Basal medium | |
|---|---|---|
| | A | B |
| Peptone | 10 | |
| Proteose peptone No. 3 | | 10 |
| Meat extract | 10 | 10 |
| Yeast extract | 3 | 5 |
| Polysorbate 80 | | 1 |
| Magnesium sulfate | | 0.1 |
| Manganese sulfate | | 0.05 |
| Ammonium citrate | | 2 |
| Sodium chloride | 5 | |
| Sodium acetate | 3 | 5 |
| L-cysteine hydrochloride | 0.5 | 0.5 |

| | | |
|---|---|---|
| Unit: g/1,000 mL | | |

**[Table 2]**

| | Basal medium | | Sugar source (final conc. 1%) | Viable cell count | Colony diameter (mm) |
|---|---|---|---|---|---|
| | Type | Concentration (-fold) | | | |
| 1-1* | Basal medium A | 0.1 | D-mannitol | <5.0 × 10⁹ cells/g | <0.1 |
| 1-2* | Basal medium A | 0.1 | D-sorbitol | <5.0 × 10⁹ cells/g | <0.1 |
| 1-3* | Basal medium A | 0.3 | D-mannitol | 1.6 × 10¹¹ cells/g | 0.5 |
| 1-4* | Basal medium A | 0.3 | D-sorbitol | 1.4 × 10¹¹ cells/g | 0.5 |
| 1-5 | Basal medium A | 0.6 | D-mannitol | 1.8 × 10¹¹ cells/g | 0.8 |
| 1-6 | Basal medium A | 0.6 | D-sorbitol | 1.6 × 10¹¹ cells/g | 1.0 |
| 1-7 | Basal medium A | 1.0 | D-mannitol | 1.5 × 10¹¹ cells/g | 1.3 |
| 1-8 | Basal medium A | 1.0 | D-sorbitol | 1.4 × 10¹¹ cells/g | 1.3 |
| 1-9* | Basal medium A | 1.5 | D-mannitol | 1.5 × 10¹¹ cells/g | 1.3 |
| 1-10* | Basal medium A | 1.5 | D-sorbitol | 1.7 × 10¹¹ cells/g | 1.1 |
| 1-11* | Basal medium A | 2.0 | D-mannitol | 1.5 × 10¹¹ cells/g | 0.9 |
| 1-12* | Basal medium A | 2.0 | D-sorbitol | 1.5 × 10¹¹ cells/g | 0.8 |
| 1-13* | Basal medium A | 3.0 | D-mannitol | <5.0 × 10⁹ cells/g | <0.1 |
| 1-14* | Basal medium A | 3.0 | D-sorbitol | <5.0 × 10⁹ cells/g | <0.1 |

| | | | | | |
|---|---|---|---|---|---|
| (* reference) | | | | | |

**[Table 3]**

| | Basal medium | | Sugar source (final conc. 1%) | Viable cell count | Colony diameter (mm) |
|---|---|---|---|---|---|
| | Type | Concentration (-fold) | | | |
| 2-1* | Basal medium B | 0.1 | D-mannitol | <5.0 × 10⁹ cells/g | <0.1 |
| 2-2* | Basal medium B | 0.1 | D-sorbitol | <5.0 × 10⁹ cells/g | <0.1 |
| 2-3* | Basal medium B | 0.3 | D-mannitol | 1.7 × 10¹¹ cells/g | 0.8 |
| 2-4* | Basal medium B | 0.3 | D-sorbitol | 1.6 × 10¹¹ cells/g | 0.6 |
| 2-5 | Basal medium B | 0.6 | D-mannitol | 1.7 × 10¹¹ cells/g | 1.0 |
| 2-6 | Basal medium B | 0.6 | D-sorbitol | 1.7 × 10¹¹ cells/g | 0.9 |
| 2-7* | Basal medium B | 1.0 | D-mannitol | 1.7 × 10¹¹ cells/g | 1.0 |
| 2-8* | Basal medium B | 1.0 | D-sorbitol | 1.9 × 10¹¹ cells/g | 1.1 |

| | | | | | |
|---|---|---|---|---|---|
| (* reference) | | | | | |

As indicated in Table 2, amongst the culture media 1-1 to 1-14 based on the same composition as that of the basal medium A, the viable cell count was less than 5.0 × 10⁹ cells/g in those cases where bacteria were cultured in the culture media 1-1 and 1-2 where the concentrations were 0.1-fold and in the culture media 1-13 and 1-14 where the concentrations were 3.0-fold.

In addition, the viable cell counts were 1.6 × 10¹¹ cells/g and 1.4 × 10¹¹ cells/g, respectively, in those cases where bacteria were cultured in the culture media 1-3 and 1-4 where the concentrations were 0.3-fold, although the formed colonies were relatively small with a colony diameter of 0.5 mm.

On the other hand, in those cases where bacteria were cultured in other culture media (i.e., culture media 1-5 to 1-12), the viable cell counts were from 1.4 × 10¹¹ cells/g to 1.8 × 10¹¹ cells/g, and favorable colony growth was observed with a colony diameter of 0.8 to 1.3 mm.

Accordingly, it was found that by using the same composition as that of the basal medium A within the concentration range from 0.6 to 2.0-fold as the basal medium, the viable cell counts increased, and the colonies with a colony diameter of 0.8 mm or larger which were easily identifiable were formed.

As indicated in Table 3, amongst the culture media 2-1 to 2-8 based on the same composition as that of the basal medium B, the viable cell count was less than 5.0 × 10⁹ cells/g in those cases where bacteria were cultured in the culture media 2-1 and 2-2 where the concentrations were 0.1-fold.

In addition, amongst the culture media 2-3 and 2-4 where the concentrations were 0.3-fold, favorable growth was observed in those cases where bacteria were cultured in the culture medium 2-3 to which D-mannitol was added as a sugar source. On the other hand, the formed colonies were relatively small with a colony diameter of 0.6 mm in those cases where bacteria were cultured in the culture medium 2-4 to which D-sorbitol was added as a sugar source, although the viable cell count was 1.6 × 10¹¹ cells/g.

In contrast, in those cases where bacteria were cultured in other culture media (i.e., culture media 2-5 to 2-8), the viable cell counts were from 1.7 × 10¹¹ cells/g to 1.9 × 10¹¹ cells/g, and favorable colony growth was observed with a colony diameter of 0.9 to 1.1 mm.

Accordingly, it was found that by using the same composition as that of the basal medium B within the concentration range from 0.6 to 1.0-fold as the basal medium, the viable cell counts increased, and the colonies with a colony diameter of 0.9 mm or larger which were easily identifiable were formed.

From the compositions of the basal media A and B as well as the results indicated in Tables 2 and 3, a basal medium containing 6.0 to 20.0 g of peptone, 6.0 to 20.0 g of meat extract and 3.0 to 6.0 g of yeast extract within 1,000 mL of the medium was confirmed to be effective for the favorable growth of breve species.

### <Test Example 2: Study on isolation characteristics due to differences in bifidobacteria species>

As bifidobacteria, the ATCC 15700^{T} strain (breve species), the M-16V strain (breve species), the ATCC 15707^{T} strain (longum species), the BB536 strain (longum species) and the DSM 10140^{T} strain (lactis species) were used. The superscript T indicates a type strain.

Of the above strains, the M-16V strain (breve species) is deposited under the Deposition Number: BCCM/LMG23729, and the BB536 strain (longum species) is deposited under the Deposition Number: ATCC BAA-999.

The assimilation modes of breve species, infantis species, longum species and lactis species for mannitol and sorbitol which are respectively taken from Table 15.51 in Bergey's Manual of Systematic Bacteriology (1986, vol. 2, page 1,428) (hereafter, referred to as "Document A") and Bifidobacteria Microflora (vol. 1 (1), pp. 3-24) (hereafter, referred to as "Document B") are shown in Table 4.

With respect to the values taken from the Document A, the letter d indicates that 11 to 89% of bacterial strains exhibited assimilation mode, and the symbol "-" indicates that 90% or more bacterial strains exhibited no assimilation mode. With respect to the values taken from the Document B, the denominator values indicate the number of bacterial strains tested and the numerator values indicate the number of bacterial strains, among the bacterial strains tested, that exhibited assimilation mode.

It should be noted that although the above Bergey's Manual report the assimilation capacity of mannitol and sorbitol among 11 to 89% of breve species, according to the Document B, all bacterial strains of breve species are known to exhibit the assimilation modes for mannitol and sorbitol. In addition, it has been known that the assimilation capacity for mannitol and sorbitol is lacking in most strains of infantis species, longum species and lactis species.

**[Table 4]**

| Bacterial species | Mannitol | | Sorbitol | |
|---|---|---|---|---|
| | Doc. A | Doc. B* | Doc. A | Doc. B* |
| Breve species | d | 3/3 | d | 3/3 |
| Infantis species | - | 1/4 | - | 0/4 |
| Longum species | - | 0/2 | - | 0/2 |
| Lactis species (described as Animalis species) | - | 0/2 | - | 0/2 |

| | | | | |
|---|---|---|---|---|
| d: indicates that 11 to 89% of bacterial strains exhibited assimilation mode -: indicates that 90% or more bacterial strains exhibited no assimilation mode *: The denominator values indicate the number of bacterial strains tested and the numerator values indicate the number of bacterial strains, among the bacterial strains tested, that exhibited assimilation mode. | | | | |

The basal medium A containing 3 g of yeast extract, 10 g of meat extract, 10 g of peptone, 5 g of sodium chloride, 3 g of sodium acetate and 0.5 g of L-cysteine hydrochloride in a volume of 1,000 mL, and basal media in which the aforementioned basal medium A (having a 1.0-fold concentration) was diluted so that the concentrations of each component of the basal medium A were adjusted to those shown in Table 5 (0.1 to 0.9-fold) were prepared.

Agar was added to each of the above basal media so that the final concentration thereof (i.e., concentration following the addition of sugar sources) was 1.5% by mass, thereby preparing the respective basal media 3-1 to 3-4.

In addition, agar was added to each of the above basal media so that the final concentration thereof (i.e., concentration following the addition of sugar sources) was 1.5% by mass, relative to the total mass of the culture medium, and D-glucose, D-sorbitol or D-mannitol was further added thereto as a sugar source so that the final concentration thereof was 1% by mass, relative to the total mass of the culture medium, thereby preparing the respective culture media 3-5 to 3-16.

Each of the above bacterial strains was subcultured at 37°C for 15 hours in the mMRS culture solution by transferring the originally grown culture thereto in an amount of 3%. The culture solution in which the stable growth was confirmed was diluted with 0.85% physiological saline to an appropriate concentration.

The diluted culture solution was poured onto a plate through a pour plate method together with the culture medium, which was heated to 45°C and dissolved in advance. After the solidification of agar, incubation at 37°C under anaerobic conditions was performed for 48 ± 2 hours. Following the incubation, the diameters (mm) of the colonies that had grown in the culture medium were measured as in Test Example 1. The results are shown in Table 5.

In Table 5, the expression "N. T." indicates that no test (namely, incubation and measurement of colony diameters) has been carried out.

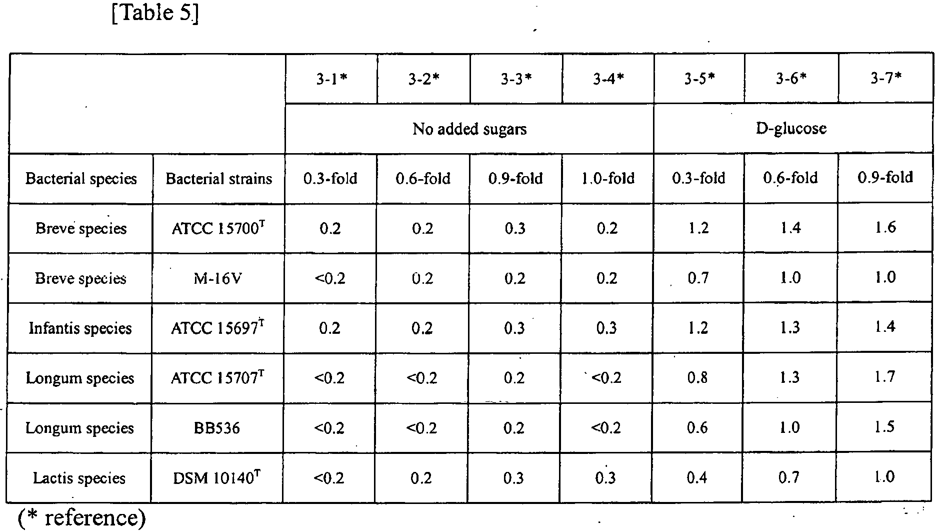

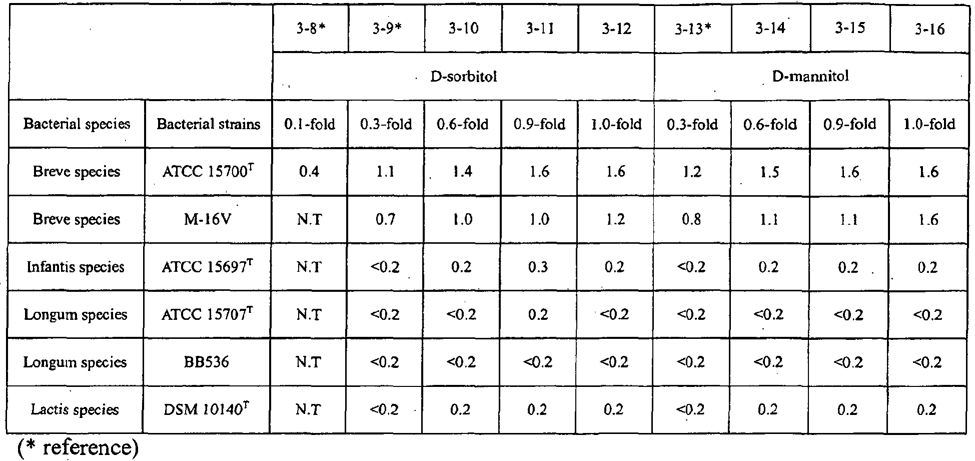

As shown in Table 5, among all bacterial strains tested, only colonies not larger than 0.3 mm grew even when incubated with the culture media (3-1 to 3-4) where no sugar source was added.

In those cases where the culture media (3-5 to 3-7) to which 1% by mass of D-glucose was added were used, the diameter of the formed colonies increased proportional to the concentrations of the components of culture media. In the culture media where the concentrations of the medium components were 0.6-fold or higher, colonies equal to or larger than 0.7 mm in diameter were formed, regardless of the bacterial species, although no difference was observed among the bacterial species.

In those cases where the culture media (3-8 to 3-12) to which 1% by mass of D-sorbitol was added were used, both the ATCC 15700^{T} strain and the M-16V strain of breve species formed colonies not smaller than 0.7 mm in diameter when the concentrations of the medium components were from 0.3-fold to 1.0-fold.

In contrast, all the colonies formed by four bacterial strains of infantis species, longum species and lactis species other than breve species were not larger than 0.3 mm in diameter, and thus it became clear that these bacterial strains only grew colonies that were easily distinguishable from the colonies of breve species in terms of size.

From these results, it was confirmed that by using a culture medium in which a predetermined amount of D-sorbitol or D-mannitol was added to a specific basal medium as a sole sugar source, it is possible to increase the size of the colonies formed only by the breve species, and to easily determine the viable cell count thereof.

Therefore, even if the infantis species, longum species, lactis species or the like other than the breve species were also present among the bacteria to be tested, it is possible to identify the breve species alone and to easily determine the viable cell count thereof through the incubation using the above culture medium.

### <Example 1, Comparative Examples 1 and 2>

A milk powder product known to contain both the bacteria powder of breve sp. M-16V strain and the bacteria powder of longum sp. BB 536 strain was prepared.

The viable cell count for each bacterial species within the aforementioned milk powder product was determined in the same manner as in Test Example 1 using three types of culture media; i.e., a BL agar medium containing sterile defibrinated horse blood (a conventional culture medium on which colonies were regarded as distinguishable from the shapes thereof), the RCA medium (a culture medium in which both two strains of bifidobacteria were known to form colonies), and a culture medium prepared by adding D-sorbitol to the following basal medium to a concentration of 1% by mass (namely, the culture medium of the present invention). However, the incubation was carried out, only with the BL agar medium, by the spread method not the pour plate method.

Basal medium: a basal medium containing 2.5 g of yeast extract, 8.2 g of meat extract, 8.2 g of peptone, 4.1 g of sodium chloride, 4.1 g of sodium acetate, 0.41 g of L-cysteine hydrochloride and 15 g of agar in 1,000 mL of the culture medium.

In an example where the BL medium was used, the breve species and the longum species were counted separately based on the shape and microscopic visualization of the colonies grown on the BL medium. The combined total of these values are shown as the viable cell count for two species put together.

In the examples where the RCA medium and the culture medium of the present invention were used, the viable cell count was determined from the number of colonies obtained by growth.

In addition, in Table 6, the symbol "-" indicates that no count was made since the size of the colonies was less than 0.2 mm. Further, in Table 6, the expression "%" denotes "% by mass".

**[Table 6]**

| | Ex. 1 | Comp. Ex. 1 | Comp. Ex. 2 |
|---|---|---|---|
| Culture medium | Culture medium of the present invention | BL medium (spread method) | RCA medium |
| Sugar | 2% D-sorbitol | 1% D-glucose | 0.5% D-glucose |
| | | | 0.1% soluble starch |
| Breve species | 9.1 × 10⁶ cells/g | 6.7 × 10⁶ cells/g | - |
| Longum species | - | 1.5 × 10⁷ cells/g | - |
| Two species in total | - | 2.2 × 10⁷ cells/g | 2.3 × 10⁷ cells/g |

As indicated in the results shown in Table 6, the viable cell count measured using the culture medium of the present invention was equivalent to or more than the viable cell count of breve species measured using the BL medium containing sterile defibrinated horse blood. Therefore, it was demonstrated that it was possible to identify the breve species alone and to determine the viable cell count thereof by using the culture medium of the present invention, and the obtained viable cell count was also almost as accurate as the value when the aforementioned BL medium was used.

On the other hand, the viable cell count measured using the RCA medium was equivalent to the viable cell count of breve species and longum species put together measured using the BL medium containing sterile defibrinated horse blood. Accordingly, when the RCA medium was used, it was impossible to distinguish the viable cell count of breve species from that of longum species.

### [Industrial Applicability]

According to the present invention, there are provided a measurement method capable of easily measuring the viable cell count of breve species alone, amongst the bacterial cells tested which include bifidobacteria, by using a specific culture method; and a culture medium which is useful as a selective medium for the above measurement method and which is also easy to prepare.

## Claims

1. A method comprising:
measuring a viable cell count of a microorganism belonging to Bifidobacterium breve alone, amongst bacteria to be tested which include a microorganism belonging to the genus Bifidobacterium, through use of a culture medium,
wherein said culture medium meets the following requirements 1) and 2), and said viable cell count is determined by measuring colonies having a diameter of 0.7 mm or larger which are formed in said culture medium:
1) contains at least one sugar alcohol selected from sorbitol and mannitol as a sole sugar source, wherein a concentration of said sugar alcohol within said culture medium is not more than 4% by mass, relative to the total mass of the culture medium; and
2) contains peptone, meat extract and yeast extract as nitrogen sources,
wherein a content of said peptone is 6.0 to 14.0 g/1,000 mL,
a content of said meat extract is 6.0 to 14.0 g/1,000 mL, and
a content of said yeast extract is 1.8 to 4.2 g/1,000 mL,
in 1,000 mL of said culture medium.

2. The method according to Claim 1, wherein said microorganism belonging to Bifidobacterium breve is a Bifidobacterium breve M-16V strain.

3. The method according to Claim 1 or 2,
wherein said bacteria to be tested include, as said microorganism belonging to the genus Bifidobacterium,
said microorganism belonging to Bifidobacterium breve, and
at least one type of microorganism selected from the group consisting of a microorganism belonging to Bifidobacterium longum subsp. longum, a microorganism belonging to Bifidobacterium animalis subsp. lactis, and a microorganism belonging to Bifidobacterium longum subsp. infantis.

4. The method according to Claim 3, wherein said microorganism belonging to Bifidobacterium longum subsp. longum is a Bifidobacterium longum subsp. longum BB536 strain.

5. A culture medium for measuring a viable cell count of a microorganism belonging to Bifidobacterium breve alone, amongst bacteria to be tested which include a microorganism belonging to the genus Bifidobacterium, which is used in the method described in any one of Claims 1 to 4, wherein said culture medium satisfies the following requirements 1) to 5):
1) contains at least one sugar alcohol selected from sorbitol and mannitol as a sole sugar source;
2) a concentration of said sugar alcohol is not more than 4% by mass, relative to the total mass of the culture medium;
3) contains yeast extract, within 1,000 mL of the culture medium, at a concentration from 1.8 to 4.2 g/1,000 mL;
4) contains meat extract, within 1,000 mL of the culture medium, at a concentration from 6.0 to 14.0 g/1,000 mL; and
5) contains peptone, within 1,000 mL of the culture medium, at a concentration from 6.0 to 14.0 g/1,000 mL.

## Patentansprüche

1. Ein Verfahren, umfassend:
Messen einer Anzahl lebensfähiger Zellen ausschließlich eines zu Bifidobacterium breve gehörenden Mikroorganismus unter zu untersuchenden Bakterien, welche einen Mikroorganismus einschließen, der zur Gattung Bifidobacterium gehört, unter Verwendung eines Kulturmediums,
wobei das Kulturmedium die folgenden Bedingungen 1) und 2) erfüllt, und die Anzahl der lebensfähigen Zellen durch Messen von Kolonien mit einem Durchmesser von 0,7 mm oder größer, welche in dem Kulturmedium gebildet werden, bestimmt wird:
1) enthält mindestens einen Zuckeralkohol, ausgewählt aus Sorbitol und Mannitol, als einzige Zuckerquelle, wobei eine Konzentration des Zuckeralkohols innerhalb des Kulturmediums nicht mehr als 4 Massen%, bezogen auf die Gesamtmasse des Kulturmediums, beträgt; und
2) enthält Pepton, Fleischextrakt und Hefeextrakt als Stickstoffquellen,
wobei ein Gehalt des Peptons 6,0 bis 14,0 g/1.000 ml beträgt,
ein Gehalt des Fleischextrakts 6,0 bis 14,0 g/1.000 ml beträgt und
ein Gehalt des Hefeextrakts 1,8 bis 4,2 g/1.000 ml beträgt,
in 1.000 ml des Kulturmediums.

2. Das Verfahren nach Anspruch 1, wobei der zu Bifidobacterium breve gehörende Mikroorganismus ein Bifidobacterium breve M-16V-Stamm ist.

3. Das Verfahren nach Anspruch 1 oder 2,
wobei die zu untersuchenden Bakterien als den zur Gattung Bifidobacterium gehörenden Mikroorganismus einschließen:
den zu Bifidobacterium breve gehörenden Mikroorganismus und
mindestens eine Art von Mikroorganismus, ausgewählt aus der Gruppe bestehend aus einem zu Bifidobacterium longum subsp. longum gehörenden Mikroorganismus, einem zu Bifidobacterium animalis subsp. lactis gehörenden Mikroorganismus und einem zu Bifidobacterium longum subsp. infantis gehörenden Mikroorganismus.

4. Das Verfahren nach Anspruch 3, wobei der zu Bifidobacterium longum subsp. longum gehörende Mikroorganismus ein Bifidobacterium longum subsp. longum BB536-Stamm ist.

5. Ein Kulturmedium zur Messung einer Anzahl lebensfähiger Zellen ausschließlich eines zu Bifidobacterium breve gehörenden Mikroorganismus unter zu untersuchenden Bakterien, welche einen Mikroorganismus einschließen, der zur Gattung Bifidobacterium gehört, welches in dem Verfahren verwendet wird, das in einem der Ansprüche 1 bis 4 beschrieben ist, wobei das Kulturmedium die folgenden Bedingungen 1) bis 5) erfüllt:
1) enthält mindestens einen Zuckeralkohol, ausgewählt aus Sorbitol und Mannitol, als einzige Zuckerquelle;
2) eine Konzentration des Zuckeralkohols beträgt nicht mehr als 4 Massen%, bezogen auf die Gesamtmasse des Kulturmediums;
3) enthält Hefeextrakt in 1.000 ml des Kulturmediums in einer Konzentration von 1,8 bis 4,2 g/1.000 ml;
4) enthält Fleischextrakt in 1.000 ml des Kulturmediums in einer Konzentration von 6,0 bis 14,0 g/1.000 ml; und
5) enthält Pepton in 1.000 ml des Kulturmediums in einer Konzentration von 6,0 bis 14,0 g/1.000 ml.

## Revendications

1. Procédé consistant à :
mesurer un nombre de cellules viables d'un microorganisme appartenant à Bifidobacterium breve uniquement, parmi les bactéries à tester qui comprennent un microorganisme appartenant au genre Bifidobacterium, en utilisant un milieu de culture, dans lequel ledit milieu de culture satisfait les exigences 1) et 2) suivantes, et ledit nombre de cellules viables est déterminé en mesurant des colonies ayant un diamètre supérieur ou égal à 0,7 mm qui se sont formées dans ledit milieu de culture :
1) contient au moins un alcool de sucre choisi parmi le sorbitol et le mannitol en tant qu'une source de sucre unique, dans lequel une concentration dudit alcool de sucre dans ledit milieu de culture n'est pas supérieure à 4 % en poids, par rapport au poids total du milieu de culture ; et
2) contient une peptone, un extrait de viande et un extrait de levure en tant que sources d'azote,
dans lequel une teneur en ladite peptone est de 6,0 à 14,0 g/1000 ml,
une teneur en ledit extrait de viande est de 6,0 à 14,0 g/1000 ml, et
une teneur en ledit extrait de levure est de 1,8 à 4,2 g/1000 ml,
dans 1000 ml dudit milieu de culture.

2. Procédé selon la revendication 1, dans lequel ledit microorganisme appartenant à Bifidobacterium breve est une souche M-16V de Bifidobacterium breve.

3. Procédé selon la revendication 1 ou 2,
dans lequel lesdites bactéries à tester comprennent, en tant que ledit microorganisme appartenant au genre Bifidobacterium:
ledit microorganisme appartenant à Bifidobacterium breve, et
au moins un type de microorganisme choisi dans le groupe constitué par un microorganisme appartenant à Bifidobacterium longum subsp. longum, un microorganisme appartenant à Bifidobacterium animalis subsp. lactis et un microorganisme appartenant à Bifidobacterium longum subsp. infantis.

4. Procédé selon la revendication 3, dans lequel ledit un microorganisme appartenant à Bifidobacterium longum subsp. longum est une souche BB536 de Bifidobacterium longum subsp. longum.

5. Milieu de culture pour la mesure d'un nombre de cellules viables d'un microorganisme appartenant à Bifidobacterium breve uniquement, parmi les bactéries à tester qui comprennent un microorganisme appartenant au genre Bifidobacterium, qui est utilisé dans le procédé décrit selon l'une quelconques des revendications 1 à 4, dans lequel ledit milieu de culture satisfait les exigences 1) à 5) suivantes :
1) contient au moins un alcool de sucre choisi parmi le sorbitol et le mannitol en tant qu'une source de sucre unique ;
2) une concentration dudit alcool de sucre n'est pas supérieure à 4 % en poids, par rapport au poids total du milieu de culture ;
3) contient un extrait de levure, dans 1000 ml du milieu de culture, à une concentration de 1,8 à 4,2 g/1000 ml ;
4) contient un extrait de viande, dans 1000 ml du milieu de culture, à une concentration de 6,0 à 14,0 g/1000 ml ; et
5) contient une peptone, dans 1000 ml du milieu de culture, à une concentration de 6,0 à 14,0 g/1000 ml.
